# EUROPÄISCHE PATENTSCHRIFT

(11) **EP 1 383 552 B1**
(45) Veröffentlichungstag und Bekanntmachung des Hinweises auf die Patenterteilung: **22.02.2006**
(21) Anmeldenummer: 02726083.5
(22) Anmeldetag: 03.05.2002
(51) Int. Cl.: A61L 31/02, A61L 31/10

(54) **GEWEBE ZUM CHIRURGISCHEN VERSCHLUSS VON HERNIEN**
MATERIAL FOR SURGICALLY CLOSING HERNIAS
TISSU POUR L'OBTURATION CHIRURGICALE DES HERNIES

(30) Priorität: 03.05.2001 DE 10121623
(43) Veröffentlichungstag der Anmeldung: 28.01.2004
(73) Patentinhaber: Richter, Horst A., 52146 Würselen (DE); Siegel, Rolf, Dr. med., 97076 Würzburg (DE)
(72) Erfinder: Richter, Horst A., 52146 Würselen (DE); Siegel, Rolf, Dr. med., 97076 Würzburg (DE)
(74) Vertreter: Priesmeyer, Thomas
(86) Internationale Anmeldenummer: PCT/DE2002/001605
(87) Internationale Veröffentlichungsnummer: WO 2002/089867

(56) Entgegenhaltungen:
- EP-A- 0 905 101
- FR-A- 2 735 353
- US-A- 5 728 157

## Beschreibung

Bei der Erfindung handelt es sich um ein künstliches Gewebe zum chirurgischen Verschluß von Hernien, wie beispielsweise von Zwerchfell-, Nabel-, Leisten- oder auch Narbenbrüchen. Zum Stand der Technik und der gegenwärtigen Diskussion, siehe, beispielsweise: R. Flöhl: Kunststoffnetze verhindern Narbenbrüche, Frankfurter Allgemeine Zeitung vom 10. Mai 2000 sowie V. Schumpelick et al.: Meshes in der Bauchwand, Der Chirurg (1999) 70, S. 876 - 887.

Hieraus geht hervor, daß die nicht-resorbierbaren Materialien, aus denen die bislang eingesetzten Netze bestehen, wie zum Beispiel Polypropylen PP (Marlex®), Polytetrafluorethylen PTFE (GoreTex®) und Polyethylenterephthalat PET (Mersilene®), aufgrund ihrer inhärenten Materialeigenschaften und mangelnden Biokompatibilität, was sich durch chronische Entzündungsreaktionen äußert, nicht befriedigen.

Aufgabe der Erfindung ist es, ein nicht-resorbierbares Gewebe, so auch ein Netz, zu schaffen, daß durch geeignete Materialauswahl und Weiterverarbeitung in bezug auf Hydrolysestabilität, Drapierbarkeit, Flexibilität, Steifigkeit und Shore-Härte in vielfältiger Weise ausgestaltet werden kann und daß eine biokompatible, insbesondere cytokompatible Oberfläche besitzt.

Die Aufgabe wird dadurch gelöst, daß man ein Gewebe aus Glasfaser einsetzt, dessen Oberfläche mit Silikon beschichtet ist, wobei auf die Silikonoberfläche eine oder mehrere biologisch aktive Substanzen ionogen oder kovalent gebunden sind.

Gewebe aus Glasfaser, die für den Anwendungszweck geeignet sind, sind bekannt bzw. können mit bekannten Materialien und Verarbeitungstechnologien leicht geschaffen werden. Siehe hierzu, beispielsweise, Firmenprospekte der Firmen cs-Interglas, D-89155 Erbach über "Garne für Interglasgewebe" und "Gewebe für die Kunststoffverstärkung".

Bevorzugt werden zur Herstellung der erfindungsgemäßen Gewebe hydrolysestabile Glassorten eingesetzt, ganz besonders bevorzugt C-Glas oder Quarzglas. Zur Herstellung der Gewebe werden vorzugsweise Garntypen ausgewählt, bei denen die vollständige, "lunkerfreie" Beschichtung der hier vorliegenden, ca. 3 bis 20 µm starken, Spinnfäden mit Silikon ohne großen apparativen Aufwand möglich ist, besonders bevorzugt werden als Garntypen gefachtes Garn, Roving, Stapelfasergarn und texturiertes Gam eingesetzt. Besonders bevorzugt werden Game mit Textilschlichte, die thermisch oder wässrig wieder entfernt werden kann, eingesetzt.

Die Verarbeitung derartiger Game bzw. entsprechender Fäden zu Geweben ist Stand der Technik. Ein typisches Gewebe mißt 100 x 100 mm, hat eine Maschenweite von 0,4 mm und hat einen Fadendurchmesser von ca. 100 µm. Da das Gewebe gut drapierbar sein muß, sind die Kett- bzw- Schußfäden bevorzugt in Köperbindung und, besonders bevorzugt, in Atlasbindung (= Satin-Bindung) zueinander angeordnet.

Erfindungsgemäß ist die Oberfläche des Gewebes mit Silikon beschichtet. Silikon ist bekannt, siehe hierzu Eugene G. Rochow: Silicon and Silicones, Springer-Verlag Berlin Heidelberg 1987. Silikone, die auf Glas gut haften, werden bevorzugt eingesetzt; ganz besonders bevorzugt, werden kondensations-vemetzende Silikone eingesetzt.

Die Beschichtung des Gewebes erfolgt durch inniges-in-Kontakt-bringen des Gewebes mit Silikon, wobei angestrebt wird, daß das Silikon jeden einzelnen Spinnfaden vollständig umhüllt. Dies kann durch Verwendung von Silikonen mit niedriger, unter 10.000 mPa·s liegender, Viskosität erreicht werden oder aber dadurch, daß man höher viskose Silikone mit geeigneten Lösemitteln, z.B. Heptan, verdünnt, das Gewebe (unter Verwendung von Überdruck) mit dieser Lösung vollständig durchtränkt und anschließend das Lösemittel vollständig abdampft. Anschließend erfolgt die Aushärtung des Silikon. Die Dicke der Silikonschicht auf dem Glasfasergewebe kann dabei durch mehrmalige Wiederholung des Vorgangs frei gewählt werden. Dabei wird aber darauf geachtet, daß die Maschen des Gewebes durch das Silikon nicht verschlossen werden, so daß lebende Zellen durch dieses künstliche Gewebe hindurch wachsen können. Durch geeignete Auswahl von Silikontypen mit unterschiedlicher Shore-Härte erhält man mit Silikon beschichtetes Glasfasergewebe unterschiedlicher Steifigkeit und Flexibilität.

Zur Erhöhung der Biokompatibilität, insbesondere der Cytokompatiblität, werden auf die Oberfläche des Silikon ein oder mehrere biologisch aktive Substanzen ionogen und/oder kovalent gebunden. Verfahren zur Immobilisierung derartiger Substanzen sind bekannt, bevorzugt werden naßchemische Verfahren eingesetzt. Als biologisch aktive Substanz wird bevorzugt Kollagen, da es Zelladhärenz und Zellvermehrung von Fibroblasten fördert, auf die Oberfläche ionogen gebunden.

### Beispiel:

Eingesetzt wird ein 100 x 100 mm messendes Glasfasergewebe aus C-Glas. Die nominale Maschenweite beträgt 265 µm, die nominale Fadenstärke 120 µm. Die Textilschlichte des Glasfasergewebes wird durch thermische Behandlung, 12 Stunden bei 400°C, entfemt. Anschließend wird das Gewebe mehrmals mit 2M HCl und abschließend mit de-ionisiertem Wasser gewaschen.

Die Beschichtung mit Silikon erfolgt dadurch, daß man eine 1%ige (w/v) Dispersion von Medical Adhesive Silicone Type A in Heptan über Nacht bei 5 bar Überdruck auf das trockene Glasfasemetz einwirken läßt. Danach läßt man das Heptan abdampfen und wiederholt nach 24 Stunden den Vorgang erneut. Insgesamt wird das Glasfasernetz dreimal mit Silikondispersion kontaktiert. Die entgültige Aushärtung des Silikon erfolgt über 10 Tage bei Raumtemperatur und ca. 70%iger relativer Luftfeuchte.

Zur Bindung biologisch aktiver Substanzen wird das derart mit Silikon beschichtete Glasfasergewebe für 12 min mit einer 5,7 gew.-%igen KOH-Lösung in Methanol behandelt und anschließend mit de-ionisiertem Wasser gewaschen. Als biologisch aktive Substanz wird Kollagen dadurch auf die derart aktivierte Silikonoberfläche gebunden, daß man das mit Silikon beschichtete Glasfasergewebe mit einer 0,5 gew.-%igen Kollagen-Lösung in Wasser, pH4, über Nacht in innigen Kontakt bringt.

Das nunmehr mit Kollagen beschichtete silikonisierte Glasfasergewebe wird dann gewaschen und durch Autoklavieren sterilisiert und steht dann (im feuchten Zustand) zur Implantation zur Verfügung.

## Patentansprüche

1. Gewebe zum chirurgischen Verschluß von Hernien, **dadurch gekennzeichnet, daß** es sich um ein Gewebe aus Glasfaser handelt, dessen Oberfläche mit Silikon beschichtet ist, wobei auf die Silikonoberfläche eine oder mehrere biologisch aktive Substanzen ionogen oder kovalent gebunden sind.

## Claims

1. A fabric for surgically suturing hernias, **characterized in that** it concerns a fabric made of glass fibers whose surface is coated with silicone, with one or several biologically active substances being bound to the silicone surface in an ionogenic or covalent manner.

## Revendications

1. Tissu destiné à fermer par intervention chirurgicale une hernie, **caractérisé en ce qu'**il s'agit d'un tissu en fibres de verre sur la surface revêtue de silicone duquel une ou plusieurs substances biologiquement actives sont liées par une liaison ionogène ou covalente.
